# DEMANDE DE BREVET EUROPEEN

(11) **EP 4 706 386 A2**
(43) Date de publication de la demande: **11.03.2026**
(21) Numéro de dépôt: 25225003.0
(22) Date de dépôt: 17.12.2021
(51) Int. Cl.: A01K 27/00, A61K 9/70, A61K 47/02, A61K 47/32, A01K 13/00, A01K 15/00, A61K 35/02, A61K 35/12, A61D 7/00, A61K 33/06, A61K 9/42, A61K 31/74, A01N 25/18, A01N 25/08

(54) **DISPOSITIFS PORTABLES POUR ANIMAUX DE COMPAGNIE AVEC DIFFUSION PASSIVE DE COMPOSITIONS SEMIOCHIMIQUES**

(30) Priorité: 21.12.2020 FR 2013842
(62) Demande divisionnaire de: 21839548.1
(71) Demandeur: VIRBAC, 06516 Carros (FR)
(72) Inventeur: GIRARDIN, Aurélie, 06700 Saint Laurent du Var (FR); MONGINOUX, Patricia, 06270 VILLENEUVE LOUBET (FR); LE JEANNE, Cécile, 87360 Lussac les Eglises (FR); JOLIVET, Jean, 36310 Chaillac (FR)
(74) Mandataire: Gevers & Orès

(57) **Abrégé**

La présente invention a pour objet des dispositifs portables pour animaux de compagnie capables de diffuser passivement des compositions sémiochimiques ; ces dispositifs apaisants permettent aux animaux de mieux gérer les situations génératrices de stress (situations de séparation, sevrage, transport, peur des bruits, des feux d'artifice etc.) et leur permettre de mieux s'adapter aux situations nouvelles.

## Description

La présente invention a pour objet des dispositifs portables pour animaux de compagnie capables de diffuser passivement des compositions sémiochimiques.

La présente invention a en particulier pour objet des dispositifs capables de diffuser des compositions sémiochimiques pendant une durée supérieure aux dispositifs connus de l'art antérieur.

On connait dans l'art antérieur des dispositifs capables de diffuser passivement des compositions sémiochimiques. Les compositions sémiochimiques sont des composés ou des mélanges de composés naturellement sécrétés par les animaux pour communiquer au sein d'une même espèce. Par exemple, la chienne sécrète pendant la lactation une phéromone pour rassurer ses chiots lorsqu'ils sont près d'elle. De la même façon, le chat sécrète, au niveau de sa face, des phéromones faciales qu'il dépose dans les endroits connus pour marquer un territoire familier.

De nombreuses études ont été réalisées pour identifier ces mélanges de composés et les reproduire synthétiquement.

De tels mélanges de composés sont par exemple indiqués pour aider les chiens à mieux gérer les situations génératrices de stress (situations de séparation, sevrage, transport, peur des bruits, des feux d'artifice etc.) et leur permettre de mieux s'adapter aux situations nouvelles.

Les produits concernés sont composés en particulier de sprays, de diffuseurs actifs (prises électriques chauffant une composition sémiochimique dispersée ou solubilisée dans un solvant organique) et de colliers.

Les colliers de l'art antérieur, en particulier ceux de la marque ADAPTIL^{®}, dont le principe actif ou actif sémiochimique est l'analogue synthétique de la phéromone sécrétée par la chienne pour rassurer ses chiots à son contact, contiennent un polymère, l'éthylène-acétate de vinyle ou EVA, et une composition sémiochimique constituée d'un mélange d'esters méthyliques d'acides gras. La composition sémiochimique, imprégnée dans le polymère, diffuse passivement dans l'air où elle est perçue par l'organe voméro-nasal du chien.

La vitesse de diffusion de ce collier (ou vitesse de relargage) est telle que le collier peut être utilisé pendant quatre semaines selon le fabricant. La Demanderesse a pu mettre en évidence qu'après quatre semaines, seule une fraction mineure de la composition sémiochimique avait effectivement diffusé hors du dispositif. Ainsi, on observe après quatre semaines qu'un maximum de 14% de la composition sémiochimique initialement présente dans le collier a diffusé.

Ces résultats indiquent de manière très claire que l'imprégnation de la composition sémiochimique dans de l'éthylène-acétate de vinyle ne permet pas la libération complète de la composition sémiochimique, et qu'après quatre semaines, la cinétique de relargage de la phéromone devient très faible, ce qui ne permet plus au dispositif d'être efficace.

La présente invention a donc pour objet l'amélioration des dispositifs de l'art antérieur, en particulier des colliers pour animaux de compagnie, contenant une composition sémiochimique.

Un premier objectif de la présente invention est de permettre une diffusion pendant une période plus longue, en particulier plus d'un mois, de la composition sémiochimique par rapport aux dispositifs de l'art antérieur.

Un deuxième objectif de la présente invention est de permettre la diffusion d'une quantité relative, par rapport à la quantité totale dans le dispositif, plus élevée de la composition sémiochimique hors du dispositif par rapport aux dispositifs de l'art antérieur.

Un troisième objectif de la présente invention est de permettre une diffusion contrôlée, c'est-à-dire la libération d'une quantité moyenne de composition sémiochimique équivalente sur successivement au moins deux intervalles de temps égaux par période de temps par rapport aux dispositifs de l'art antérieur.

Un quatrième objectif de la présente invention est de fournir un dispositif répondant de préférence au premier objectif et au deuxième objectif conservant une flexibilité suffisante pour être transformé en toutes sortes d'objets utilisables sur des animaux, en particulier sur des animaux de compagnie, en particulier des colliers.

Un cinquième objectif de la présente invention est d'obtenir un dispositif permettant une meilleure observance du traitement par le propriétaire, en particulier en augmentant la durée d'action du dispositif et en augmentant le temps entre deux applications du dispositif sur l'animal ou dans l'environnement immédiat de l'animal.

Sauf indication contraire, tous les pourcentages sont indiqués en masse d'un composant par rapport à la masse totale des composants indiqués (m/m).

Un premier objet de la présente invention est un dispositif, en particulier pour relarguer de manière continue sur un animal ou dans l'environnement immédiat d'un animal une composition sémiochimique pour animaux de compagnie, comprenant :
- une matrice polymère,
- une composition sémiochimique,
- un agent de contrôle de la diffusion de la composition sémiochimique hors du dispositif,
- éventuellement un antioxydant,
- éventuellement un agent anti-UV,
- éventuellement un agent d'écoulement.

Selon ce premier objet, la présente invention se rapporte à un dispositif pour relarguer de manière continue sur un animal ou dans l'environnement immédiat d'un animal une composition sémiochimique pour animaux de compagnie comprenant :
- une matrice polymère,
- une composition sémiochimique comprenant un mélange de dérivés volatils d'acide gras,
- un agent de contrôle de la diffusion de la composition sémiochimique hors du dispositif choisi parmi une argile, une diatomite et une zéolite.

Les dispositifs selon la présente invention sont notamment des corps façonnés solides portables par l'animal ou pouvant être installés dans l'environnement immédiat de l'animal tels que des colliers, des harnais, des pendentifs pour colliers (médaillons), des étiquettes auriculaires (*ear tag* en anglais), des colliers susceptibles d'être fixés sur des membres ou des parties du corps, des bandes adhésives et des films. Une préférence est donnée aux médaillons et aux colliers, en particulier aux colliers.

On entend par environnement immédiat de l'animal les zones où il est susceptible d'évoluer comme le foyer de son maitre, sa niche, un véhicule, une cage de transport...

Les corps façonnés selon l'invention peuvent également prendre la forme de billes que l'utilisateur pourra disposer dans un récipient.

Les dispositifs peuvent également prendre la forme d'objets présents dans l'environnement de l'animal comme des gamelles, des tapis de sol, des couvertures, des vêtements ou des tapis de dressage.

La taille et la forme du dispositif, quel qu'il soit, est adapté à l'animal auquel il est destiné ; le dispositif est équipé si nécessaire d'un moyen de fixation connu de l'homme du métier. Par exemple, concernant les systèmes de fermeture des colliers constitué d'une bande extrudée, l'attache peut être en plastique moulée avec son rivet, fixée à l'aide d'un rivet métallique, fixée avec une agrafe métallique, ou bien être un système de boucle métallique avec des passants maintenus dans la lanière repliée grâce à plusieurs rivets.

Dans le cas des colliers, il est aussi envisageable de proposer des colliers d'une longueur standard qui seront coupés par le propriétaire de l'animal pour l'ajuster au mieux à la taille du cou de l'animal cible, par exemple, un chiot, un petit chien, un chien moyen ou un grand chien.

Dans un mode de réalisation, le dispositif selon l'invention est un collier d'une longueur prédéterminée sur lequel est fixé un passant permettant de maintenir la longueur du collier en excès contre la partie du collier en contact avec le cou de l'animal. Le passant permet d'une part de conserver l'intégralité de la longueur du collier dans lequel est présente la composition sémiochimique et d'autre part que cet excès de longueur ne gêne pas l'animal ou le propriétaire.

Le dispositif peut avoir une surface lisse ou bien présenter des reliefs qui augmentent la surface d'échange et la quantité d'actifs sémiochimiques libérés en un temps donné.

Dans le cadre de la présente invention, le dispositif est donc un composite comprenant une matrice polymère en tant que phase continue dans laquelle est dispersé un agent de contrôle de la diffusion de la composition sémiochimique hors du dispositif. Les termes « composites » et « matrice polymère » s'entendent au sens de la présente invention dans leur sens commun pour l'Homme du Métier (par ex. défini dans « *Compendium-of-Polymer-Terminology-and-Nomenclature-IUPAC-Recommendations-2008 »).* Le terme « matrice polymère » désigne une matrice comprenant un polymère de structure continue, non mousseuse ou alvéolée et exclut donc les mousses polymères et les matériaux polymériques ayant une structure alvéolaire ou cellulaire.

La matrice polymère doit posséder une résistance et une souplesse suffisantes pour garantir que le dispositif ne se rompe pas ou ne devienne pas cassant pendant sa fabrication et son utilisation. La matrice polymère doit également avoir une durabilité suffisante pour résister à l'usure normale lorsque le dispositif est portée par, ou appliqué à, l'animal. De plus, la matrice polymère doit permettre une migration adéquate, c'est-à-dire la diffusion passive de la composition sémiochimique jusqu'à la surface du dispositif ainsi que le relargage de ladite composition sur l'animal ou dans l'environnement de l'animal, en particulier de son appareil olfactif.

On utilisera avantageusement des polymères thermoplastiques, des élastomères et des résines polyvinyliques thermoplastiques. Les polyoléfines thermoplastiques et flexibles et les élastomères sont appropriés pour une utilisation en tant que substance support ou base pour les corps façonnés. Parmi ceux-ci, on peut citer les résines polyvinyliques, l'EPDM (terpolymère éthylène/propylène-diène), le polyéthylène (HDPE ou LLDPE par exemple) et le polypropylène, suffisamment compatibles avec les composés actifs précités.

Les résines polyvinyliques comprennent des polyhalogénures de vinyle, tels que le chlorure de polyvinyle, le chlorure de polyvinyle-acétate de vinyle et le fluorure de polyvinyle ; les polyvinylbenzènes, tels que le polystyrène et le polyvinyltoluène et les copolymères d'acétate de vinyle, tels que le copolymère thermoplastique d'acétate de vinyle et d'éthylène ou EVA.

D'autres matières plastiques qui conviennent pour une utilisation comme matrice pour les dispositifs selon l'invention sont des élastomères thermoplastiques. On peut par exemple citer les polyoléfines thermoplastiques (TPO).

De manière avantageuse, on emploie les résines polyvinyliques citées ci-dessus. De manière préférée, on emploiera les copolymères thermoplastiques d'acétate de vinyle et d'éthylène (EVA) ; les propriétés, notamment de souplesse et de résistance, de ces copolymères varient selon leur teneur en acétate de vinyle (on parle aussi de grade), de préférence, on utilisera des copolymères thermoplastiques d'acétate de vinyle et d'éthylène ayant une teneur en acétate de vinyle comprise entre 5 et 20%, de préférence, entre 5 et 16%, encore préférentiellement de l'ordre de 9%. Ces grades d'EVA sont particulièrement utiles pour la fabrication de colliers pour animaux.

La composition sémiochimique employée est de préférence une composition sémiochimique apaisante comprenant un mélange de dérivés volatils d'acides gras.

Au sens de la présente invention, une composition sémiochimique apaisante qualifie un mélange de substances chimiques ayant valeur de signal entre des êtres vivants d'origine naturelle, par exemple isolée d'un être vivant, ou d'origine synthétique, par exemple obtenue par mélange de dérivés volatils d'un acide gras susceptible de produire un effet relaxant ou apaisant sur l'animal.

Au sens de la présente invention, on entend par « dérivé volatil d'un acide gras » tout dérivé d'un acide gras susceptible d'être diffusé sur l'animal ou dans l'environnement de l'animal sans application d'une source de chaleur d'origine électrique extérieure sur le dispositif.

Au sens de la présente invention, on entend par « acide gras » un acide monocarboxylique ou dicarboxylique à chaîne hydrocarbonée, saturé ou insaturé, linéaire ou ramifié ayant entre 4 et 22 atomes de carbones. Avantageusement, l'acide gras est choisi dans le groupe constitué de l'acide oléique, l'acide palmitique, l'acide azélaïque, l'acide pimélique, l'acide caprique, l'acide laurique, l'acide myristique, l'acide palmitoléique, l'acide linoléique, l'acide stéarique, l'acide arachidonique, l'acide n-butyrique, l'acide isobutyrique, l'acide α-méthylbutyrique, l'acide caproïque, l'acide pivalique, l'acide γ-linoléique, l'acide eicosapentanoïque, l'acide pentadécanoïque, l'acide tridécanoïque, ou l'acide docosahexanoïque.

Les dérivés volatils d'acides gras sont notamment des esters d'acides gras, tels que les esters formés à partir de l'acide gras et d'un alcool en C₁ à C₆. Avantageusement, l'ester d'acide gras est l'ester éthylique ou méthylique. De préférence, l'ester d'acide gras est l'ester méthylique d'acide gras.

De manière avantageuse, la composition sémiochimique apaisante comprend donc un mélange de dérivés volatils d'acides gras où l'acide gras est choisi dans le groupe constitué de l'acide oléique, l'acide palmitique, l'acide azélaïque, l'acide pimélique, l'acide caprique, l'acide laurique, l'acide myristique, l'acide palmitoléique, l'acide linoléique, l'acide stéarique, l'acide arachidonique, l'acide n-butyrique, l'acide isobutyrique, l'acide α-méthylbutyrique, l'acide caproïque, l'acide pivalique, l'acide γ-linoléique, l'acide eicosapentanoïque, l'acide pentadécanoïque, l'acide tridécanoïque, ou l'acide docosahexanoïque, avantageusement choisi dans le groupe constitué de l'acide oléique, l'acide palmitique, l'acide laurique, l'acide myristique, l'acide linoléique, l'acide stéarique, et l'acide pentadécanoïque.

De manière plus avantageuse, la composition sémiochimique apaisante comprend un mélange de dérivés volatils d'acides gras choisis dans le groupe constitué de l'oléate de méthyle, du palmitate de méthyle, de l'azélate de méthyle, du pimélate de méthyle, du caprate de méthyle, du laurate de méthyle, du myristate de méthyle, du palmitoléate de méthyle, du linoléate de méthyle, du stéarate de méthyle, de l'arachidonate de méthyle, du n-butyrate de méthyle, de l'isobutyrate de méthyle, de l'α-méthylbutyrate de méthyle, du caproate de méthyle, du pivalate de méthyle, du γ-linoléate de méthyle, de l'eicosapentanoate de méthyle, du pentadécanoate de méthyle, du tridécanoate de méthyle, du docosahexanoate de méthyle, avantageusement choisi dans le groupe constitué de l'oléate de méthyle, du palmitate de méthyle, du laurate de méthyle, du myristate de méthyle, du linoléate de méthyle, du stéarate de méthyle et du pentadécanoate de méthyle.

Les dérivés volatils d'acides gras et leurs proportions relatives dans la composition sémiochimique apaisante dépendent de l'animal cible.

Lorsque l'animal cible est un chien, la composition sémiochimique apaisante peut être choisie parmi celles décrites dans la demande internationale WO2009144321.

Des mélanges particuliers d'esters, de préférence d'esters méthyliques, des acides gras destiné à apaiser les chiens sont les esters des acides gras suivants :
- un mélange **A** d'acide oléique et d'acide n-butyrique ;
- un mélange **B** d'acide oléique, d'acide palmitique et d'acide linoléique ;
- un mélange **C** d'acide oléique, d'acide palmitique, d'acide linoléique et d'acide palmitoléique ;
- un mélange **D** d'acide caprique, d'acide laurique, d'acide myristique, d'acide palmitoléique, d'acide palmitique, d'acide linoléique et d'acide oléique ;
- un mélange **E** d'acide oléique, d'acide palmitique, d'acide linoléique et d'acide myristique ;
- un mélange **F** d'acide oléique, d'acide palmitique, d'acide linoléique, d'acide laurique et d'acide myristique ;
- un mélange **G** d'acide oléique, d'acide palmitique, d'acide linoléique, d'acide myristique et d'acide pentadécanoïque ;
- un mélange **H** d'acide oléique, d'acide palmitique, d'acide linoléique, d'acide myristique, d'acide pentadécanoïque et d'acide stéarique ;
- un mélange **I** d'acide oléique, d'acide palmitique, d'acide linoléique, d'acide myristique, d'acide laurique et d'acide pentadécanoïque ;
- un mélange **J** d'acide laurique, d'acide myristique, d'acide pentadécanoïque, d'acide palmitique, d'acide stéarique, d'acide oléique, et d'acide linoléique ;
- un mélange **K** d'acide oléique, d'acide azélaïque, d'acide pimélique et d'acide palmitique.

Des mélanges d'esters, de préférence les esters méthyliques, d'acides gras sont des esters des acides gras suivants :
- mélange **L** comprenant environ 55-65% d'acide oléique, et 45-35% d'acide palmitique ;
- mélange **M** comprenant environ 45% d'acide oléique, 16% d'acide azélaïque, 18% d'acide pimélique, et 21% d'acide palmitique ;
- mélange **N** comprenant environ 30% d'acide palmitique, 30% d'acide oléique, et 40% d'acide linoléique ;
- mélange **O** comprenant environ 30% d'acide palmitique, 40% d'acide linoléique, 10% d'acide palmitoléique, et 20% d'acide oléique.

Un mélange particulier **P** d'esters d'acides gras destiné à apaiser les chiens comprend environ 35% d'oléate de méthyle, environ 2% de laurate de méthyle, environ 13% de stéarate de méthyle, environ 21% de linoléate de méthyle, environ 5% de myristate de méthyle, environ 4% de pentadécanoate de méthyle, et environ 20% de palmitate de méthyle.

Les Inventeurs de la présente invention ont par ailleurs mis en évidence qu'une composition sémiochimique apaisante ne comprenant pas de pentadécanoate de méthyle conduisait à un effet similaire sur le chien à celui du mélange **P.** L'acide pentadécanoïque étant un acide gras relativement rare naturellement, dont l'origine principale est la fraction grasse du lait de vache où il est présent en quantité faible (1,2%), il est difficile d'en obtenir à un coût raisonnable. La découverte que l'absence de cet acide gras ne diminue pas l'efficacité apaisante d'une composition sémiochimique permet donc avantageusement de réduire les coûts associés à la fabrication des dispositifs selon l'invention. Un autre mélange particulier **Q** d'esters d'acides gras destiné à apaiser les chiens comprend donc entre 32% et 38% d'oléate de méthyle, entre 1 et 3% de laurate de méthyle, entre 13 et 16% de stéarate de méthyle, entre 18 et 24% de linoléate de méthyle, entre 3 et 7% de myristate de méthyle et entre 18 et 24% de palmitate de méthyle.

Un mélange plus particulier **R** d'esters d'acides gras destinés à apaiser les chiens comprend environ 35% d'oléate de méthyle, environ 2% de laurate de méthyle, environ 15,5% de stéarate de méthyle, environ 21% de linoléate de méthyle, environ 5% de myristate de méthyle et environ 21% de palmitate de méthyle.

Lorsque l'animal cible est un chat, la composition sémiochimique apaisante peut être choisie parmi celles décrites dans la demande internationale WO2015140631.

La composition sémiochimique apaisante est par exemple une phéromone apaisante pour chat qui est dérivée de sécrétions cutanées provenant de la zone située entre les omoplates et les oreilles des chats.

La composition sémiochimique destinée à apaiser les chats comprend donc avantageusement du palmitate de méthyle, du linoléate de méthyle, de l'oléate de méthyle, du stéarate de méthyle, du laurate de méthyle et du myristate de méthyle.

Un mélange particulier **S** d'esters d'acides gras destiné à apaiser les chats comprend entre 15% et 25% de palmitate de méthyle, entre 10% et 27% de linoléate de méthyle, entre 25% et 35% d'oléate de méthyle, entre 5% à 15% de stéarate de méthyle, entre 5% à 15% de laurate de méthyle et entre 5% à 15% de myristate de méthyle.

Un autre mélange particulier **T** d'esters d'acides gras destiné à apaiser les chats comprend entre 19% et 26% de palmitate de méthyle, entre 14% et 20% de linoléate de méthyle, entre 26% et 32% d'oléate de méthyle, entre 8% et 14% de stéarate de méthyle, entre 8% et 14% de laurate de méthyle et entre 8% et 14% de myristate de méthyle.

Un mélange encore plus particulier **U** comprend 22% de palmitate de méthyle, 17% de linoléate de méthyle, 28% d'oléate de méthyle, 11% méthyle stéarate, 11% de laurate de méthyle et 11% de myristate de méthyle.

Une autre composition sémiochimique pouvant être utilisée dans les dispositifs selon l'invention est décrite dans le brevet US10227321, dans laquelle au moins un dérivé volatil d'acide gras est un ester de formule selon la figure 1, dans laquelle n=0 et R est un alkyl linéaire saturé ou insaturé comprenant de 9 à 30 atomes de carbone ou n=1 et R est un alkyl linéaire saturé ou insaturé comprenant de 8 à 22 atomes de carbone.

L'animal cible est un mammifère non-humain. Il peut s'agir d'animaux de compagnie tels que les chats, les chiens, les furets et les lapins ou d'animaux d'élevage pour lesquels il est utile d'améliorer les conditions de vie tels que les lapins et les porcs. L'Homme du Métier utilisera dans le dispositif selon l'invention la composition sémiochimique capable de prévenir les manifestations de stress et/ou réduire l'anxiété adaptée à chaque espèce cible.

La quantité de composition sémiochimique dans le dispositif peut varier dans de larges proportions. La proportion de composition sémiochimique est typiquement comprise entre 0,1% et 25% en masse de la masse totale du dispositif. Avantageusement, dans un collier la composition sémiochimique représente de 1 à 10% en masse, plus avantageusement de 3 à 8% en masse, de préférence environ 5% de la masse totale du collier.

Par « environ x% », on entend au sens de la présente invention une variation de plus ou moins 0,2% par rapport au pourcentage indiqué.

Les dispositifs selon la présente invention diffèrent de ceux décrits dans l'art antérieur, en particulier du collier commercialisé sous la marque ADAPTIL^{®}, en ce qu'ils contiennent un agent de contrôle de la diffusion de la composition sémiochimique hors du dispositif capable de ralentir la diffusion de ladite composition sémiochimique.

La Demanderesse a en effet mis en évidence que l'introduction de matériaux capables d'adsorber la composition sémiochimique permet de contrôler la diffusion de ladite composition sémiochimique. Le « contrôle de la diffusion de la composition sémiochimique » s'entend comme la libération d'une quantité relative plus élevée de composition sémiochimique et/ou pendant une durée plus longue et/ou la libération d'une quantité moyenne de composition sémiochimique équivalente sur successivement au moins deux intervalles de temps égaux, par exemple, la libération d'environ 10% de la composition sémiochimique pendant une période de 14 jours pendant au moins deux périodes, par rapport au même dispositif ne contenant pas le matériau capable d'adsorber la composition sémiochimique ; ces propriétés résultent en une diffusion d'une quantité relative, par rapport à la quantité totale dans le dispositif, plus élevée de la composition sémiochimique hors du dispositif par rapport aux dispositifs de l'art antérieur.

Parmi les agents de contrôle de la diffusion de la composition sémiochimique hors du dispositif, on peut citer les matières inorganiques telles que les argiles, les diatomites et les zéolites. De manière avantageuse, on utilisera des argiles possédant une structure en chaîne (chaînes de tétraèdres de silice semblables à la hornblende), en particulier les phyllosilicates tels que la wollastonite (par exemple vendu sous la dénomination casiflux F75), la bentonite, la kaolinite, la montmorillonite, l'attapulgite, la sépiolite et la paligorskyte. De préférence, on utilise l'attapulgite, la sépiolite et la paligorskyte, encore préférentiellement, la sépiolite.

Bien que la sépiolite extraite du sol soit appropriée, on peut également utiliser des sépiolites ayant subi un traitement chimique (par exemple pour modifier son potentiel zeta) ou un traitement thermique (par exemple pour modifier sa structure).

L'agent de contrôle de la diffusion de la composition sémiochimique hors du dispositif est introduit dans le dispositif en proportions variant de 5 à 25%, avantageusement de 10 à 20%, de préférence environ 15% en masse par rapport à la masse totale du dispositif.

Les dispositifs selon la présente invention peuvent également comprendre des ingrédients conventionnellement employés pour faciliter la mise en forme du dispositif, par exemple un agent d'écoulement, un plastifiant, un antioxydant, un agent anti-UV ou tout autre ingrédient permettant d'en améliorer la stabilité dans le temps.

L'agent d'écoulement a pour rôle de faciliter l'écoulement du mélange au sein de la trémie qui alimente la vis de l'extrudeuse. Les agents d'écoulement sont bien connus de l'Homme du Métier. L'agent d'écoulement est préférentiellement choisi parmi la silice colloidale, le stéarate de magnésium, encore préférentiellement, l'agent d'écoulement est la silice colloïdale.

La stabilité des dispositifs selon la présente invention et en particulier la stabilité des composants de la composition sémiochimique, peut par exemple être améliorée en incorporant des antioxydants ou des agents anti-UV.

Parmi les antioxydants, on peut notamment citer les composés phénoliques tels que le butylhydroxyanisole (BHA), le butylhydroxytoluène (BHT), l'acide gallique, le gallate de propyle et le pyrogallol, les hydroquinones telles que la tert-butylhydroquinone (TBHQ) et la di-tert-butylhydroquinone (DTBHQ), les tocophérols, les amines telles que l'octyl butyl diphénylamine (OBPA), la poly(1,2-dihydro-2,2,4-triméthylquinoléine (Orox PK), la N,N'-phényl-p-phénylènediamine (DPD) et l'éthoxyquine, et un mélange de ces antioxydants. De tels composés sont bien connus de l'Homme du Métier et peuvent être choisis sans effort excessif ; de préférence, l'antioxydant utilisé est la TBHQ. A titre indicatif, la quantité d'antioxydants utilisée peut être comprise entre 0,001 et 0,1%.

Les agents anti-UV permettent d'opacifier le matériau du dispositif ou d'absorber les rayons UV, empêchant ainsi les rayons lumineux d'atteindre le coeur du dispositif et limitant la dégradation de ses composants par la lumière, en particulier de la composition sémiochimique. Ils peuvent être choisis parmi le noir de carbone, l'hydroxyde d'aluminium (Al(OH)3), le carbonate de calcium et les amines encombrées (en anglais *hindered amine light stabilizer* ou HALS, commercialisées par exemple sous le nom Hostavin N30P^{®}).

Selon un mode de réalisation particulier, le dispositif selon l'invention comprend en outre un agent d'écoulement tel que la silice colloïdale et un ingrédient permettant d'améliorer la stabilité dans le temps de la composition sémiochimique tels qu'un antioxydant ou un agent anti-UV.

La proportion de chacun des ingrédients dans le dispositif est choisie de manière à ce que la diffusion de la composition sémiochimique soit adaptée à l'usage souhaité tout en conservant les propriétés rhéologiques du dispositif (par exemple la flexibilité ou l'élasticité dans le cas d'un collier pour animaux).

Un deuxième objet de la présente invention concerne donc un dispositif comprenant :
- une matrice polymère,
- de 0,5 à 10% d'une composition sémiochimique telle que définie ci-dessus, de préférence environ 5%,
- de 5 à 25% d'un agent de contrôle de la diffusion de la composition sémiochimique hors du dispositif tel que défini ci-dessus, de préférence environ 15%,
- éventuellement de 0,001 à 0,1% d'un antioxydant, de préférence environ 0,005%,
- éventuellement de 0,1 à 2% d'un agent anti-UV, de préférence environ 1%,
- éventuellement de 0,1 à 5% d'un agent d'écoulement, de préférence environ 1,5%.

Dans un premier mode de réalisation, le dispositif selon la présente invention comprend :
- une matrice polymère constituée d'un copolymère thermoplastique d'acétate de vinyle et d'éthylène,
- de 0,5 à 10% d'une composition sémiochimique telle que définie ci-dessus, de préférence environ 5%,
- de 5 à 25% d'un agent de contrôle de la diffusion de la composition sémiochimique hors du dispositif tel que défini ci-dessus, de préférence environ 15%,
- éventuellement de 0,001 à 0,1% d'un antioxydant, de préférence environ 0,05%,
- éventuellement de 0,1 à 2% d'un agent anti-UV, de préférence environ 1%,
- éventuellement de 0,1 à 5% d'un agent d'écoulement, de préférence environ 1,5%.

Dans un deuxième mode de réalisation, le dispositif selon la présente invention comprend :
- une matrice polymère,
- de 0,5 à 10% d'une composition sémiochimique, de préférence environ 5%, ladite composition sémiochimique comprenant un mélange d'esters d'acides gras choisis parmi le laurate, de myristate, de palmitate, de stéarate, d'oléate et de linoléate, et éventuellement de pentadécanoate,
- de 5 à 25% d'un agent de contrôle de la diffusion de la composition sémiochimique hors du dispositif tel que défini ci-dessus, de préférence environ 15%,
- éventuellement de 0,001 à 0,1% d'un antioxydant, de préférence environ 0,05%,
- éventuellement de 0,1 à 2% d'un agent anti-UV, de préférence environ 1%,
- éventuellement de 0,1 à 5% d'un agent d'écoulement, de préférence environ 1,5%.

Dans un troisième mode de réalisation, le dispositif selon la présente invention comprend :
- une matrice polymère,
- de 0,5 à 10% d'une composition sémiochimique, de préférence environ 5%,
- de 5 à 25% d'un agent de contrôle de la diffusion de la composition sémiochimique hors du dispositif tel que défini ci-dessus, de préférence environ 15%, ledit agent étant une argile, notamment un phyllosilicate, en particulier de la sépiolite, de l'attapulgite ou de la paligorskyte,
- éventuellement de 0,001 à 0,1% d'un antioxydant, de préférence environ 0,05%
- éventuellement de 0,1 à 2% d'un agent anti-UV, de préférence environ 1%,
- éventuellement de 0,1 à 5% d'un agent d'écoulement, de préférence environ 1,5%.

Dans un mode de réalisation spécifique, le dispositif selon la présente invention comprend :
- une matrice polymère constituée d'un copolymère thermoplastique d'acétate de vinyle et d'éthylène,
- de 0,5 à 10% d'une composition sémiochimique, de préférence environ 5%, ladite composition sémiochimique comprenant un mélange d'esters d'acides gras choisis parmi le laurate, de myristate, de palmitate, de stéarate, d'oléate et de linoléate, et éventuellement de pentadécanoate,
- de 5 à 25% d'une argile, de préférence choisie parmi la sépiolite, l'attapulgite et la paligorskyte, de préférence environ 15%,
- éventuellement de 0,001 à 0,1% d'un antioxydant, de préférence environ 0,05%
- éventuellement de 0,1 à 2% d'un agent anti-UV, de préférence environ 1%,
- éventuellement de 0,1 à 5% d'un agent d'écoulement, de préférence environ 1,5%.

De manière préférée, la présente invention concerne un collier pour chien, comprenant :
- une matrice polymère constituée d'un copolymère thermoplastique d'acétate de vinyle et d'éthylène,
- environ 5% d'une composition sémiochimique, comprenant un mélange de laurate de méthyle, de myristate de méthyle, de palmitate de méthyle, de stéarate de méthyle, d'oléate de méthyle et de linoléate de méthyle, et éventuellement de pentadécanoate de méthyle,
- environ 15% de sépiolite,
- environ 0,05% d'un antioxydant,
- environ 1% d'un composé protégeant de la lumière,
- environ 1,5% d'un agent d'écoulement.

La présente invention concerne dans un troisième aspect, une méthode pour obtenir un ou plusieurs des effets suivants :
- assurer le bien-être d'un animal, dans son environnement,
- aider un animal à gérer les situations de stress et/ou d'anxiété,
- réduire le stress associé à la séparation chez un animal,
- réduire le stress associé au sevrage chez un animal,
- réduire le stress associé à la solitude chez un animal,
- réduire le stress chez un animal avant et/ou pendant un transport,
- réduire le stress associé aux bruits tels que les orages ou les feux d'artifice,
- réduire le stress associé à une pathologie, un traitement médical, la visite chez un vétérinaire pouvant nécessiter une auscultation, voire une intervention chirurgicale,
- réduire l'agressivité d'un animal,
- améliorer le comportement d'un animal, en particulier d'un animal agressif ou d'un animal peureux,
- produire un effet relaxant sur l'animal,
- aider un animal à s'adapter à une situation nouvelle par exemple au moment de l'adoption, lors de l'arrivée d'un nouvel animal ou d'un enfant dans son environnement, lors d'un déménagement,
- réduire le marquage urinaire associé au stress chez un animal,
- réduire la malpropreté et les souillures d'un animal,
- améliorer les conditions de familiarisation d'un animal avec ses congénères,
- empêcher les griffades ou la destruction du territoire,
- diminuer les manifestations sonores telles que pleurs, aboiements,
- engendrer un comportement moins agressif, plus détendu et plus affectueux notamment avec leurs maitres chez un animal,
comprenant la diffusion d'une composition sémiochimique à partir d'un dispositif tel que décrit ci-dessus, de préférence pendant une durée supérieure à 1 mois, plus avantageusement supérieure à 2 mois, de préférence pendant une durée d'environ 3 mois, l'animal étant un chien ou un chat. Ladite diffusion a lieu sur l'animal ou dans l'environnement immédiat de l'animal, c'est-à-dire proche de son appareil olfactif.

Dans un quatrième aspect, la présente invention concerne l'utilisation d'un dispositif tel que décrit ci-dessus pour obtenir un ou plusieurs des effets suivants :
- assurer le bien-être d'un animal, dans son environnement,
- aider un animal à gérer les situations de stress et/ou d'anxiété,
- réduire le stress associé à la séparation chez un animal,
- réduire le stress associé au sevrage chez un animal,
- réduire le stress associé à la solitude chez un animal,
- réduire le stress chez un animal avant et/ou pendant un transport,
- réduire le stress associé aux bruits tels que les orages ou les feux d'artifice,
- réduire le stress associé à une pathologie, un traitement médical, la visite chez un vétérinaire pouvant nécessiter une auscultation, voire une intervention chirurgicale,
- réduire l'agressivité d'un animal,
- améliorer le comportement d'un animal, en particulier d'un animal agressif ou d'un animal peureux,
- produire un effet relaxant sur l'animal,
- aider un animal à s'adapter à une situation nouvelle par exemple au moment de l'adoption, lors de l'arrivée d'un nouvel animal ou d'un enfant dans son environnement, lors d'un déménagement,
- réduire le marquage urinaire associé au stress chez un animal,
- réduire la malpropreté et les souillures d'un animal,
- améliorer les conditions de familiarisation d'un animal avec ses congénères,
- empêcher les griffades ou la destruction du territoire,
- diminuer les manifestations sonores telles que pleurs, aboiements,
- engendrer un comportement moins agressif, plus détendu et plus affectueux notamment avec leurs maitres chez un animal,
de préférence pendant une durée supérieure à 1 mois, plus avantageusement supérieure à 2 mois, de préférence pendant une durée d'environ 3 mois, l'animal étant un chien ou un chat.

La présente invention se rapporte encore à une méthode non thérapeutique pour apaiser un animal, en particulier pour :
- assurer le bien-être d'un animal, dans son environnement,
- aider un animal à gérer les situations de stress et/ou d'anxiété,
- réduire le stress associé à la séparation chez un animal,
- réduire le stress associé au sevrage chez un animal,
- réduire le stress associé à la solitude chez un animal,
- réduire le stress chez un animal avant et/ou pendant un transport,
- réduire le stress associé aux bruits tels que les orages ou les feux d'artifice,
- réduire le stress associé à une pathologie, un traitement médical, la visite chez un vétérinaire pouvant nécessiter une auscultation, voire une intervention chirurgicale,
- réduire l'agressivité d'un animal,
- améliorer le comportement d'un animal, en particulier d'un animal agressif ou d'un animal peureux,
- produire un effet relaxant sur l'animal,
- aider un animal à s'adapter à une situation nouvelle par exemple au moment de l'adoption, lors de l'arrivée d'un nouvel animal ou d'un enfant dans son environnement, lors d'un déménagement,
- réduire le marquage urinaire associé au stress chez un animal,
- réduire la malpropreté et les souillures d'un animal,
- améliorer les conditions de familiarisation d'un animal avec ses congénères,
- empêcher les griffades ou la destruction du territoire,
- diminuer les manifestations sonores telles que pleurs, aboiements,
- engendrer un comportement moins agressif, plus détendu et plus affectueux notamment avec leurs maitres chez un animal,
- faciliter le dressage et/ou l'éducation d'un animal,
comprenant l'administration d'un dispositif selon l'invention audit animal ou dans l'environnement immédiat de l'animal, c'est-à-dire proche de son appareil olfactif.

La présente invention se rapporte encore à une composition sémiochimique telle que définie ci-dessus pour prévenir les manifestations de stress et/ou réduire l'anxiété chez un animal de compagnie pendant une durée supérieure à 1 mois, plus avantageusement supérieure à 2 mois, de préférence pendant une durée d'environ 3 mois, caractérisée en ce que la composition sémiochimique est présente dans un dispositif selon l'invention.

En particulier, elle se rapporte à une composition sémiochimique telle que définie ci-dessus pour son utilisation pour apaiser un animal, en particulier pour :
- assurer le bien-être d'un animal, dans son environnement,
- aider un animal à gérer les situations de stress et/ou d'anxiété,
- réduire le stress associé à la séparation chez un animal,
- réduire le stress associé au sevrage chez un animal,
- réduire le stress associé à la solitude chez un animal,
- réduire le stress chez un animal avant et/ou pendant un transport,
- réduire le stress associé aux bruits tels que les orages ou les feux d'artifice,
- réduire le stress associé à une pathologie, un traitement médical, la visite chez un vétérinaire pouvant nécessiter une auscultation, voire une intervention chirurgicale,
- réduire l'agressivité d'un animal,
- améliorer le comportement d'un animal, en particulier d'un animal agressif ou d'un animal peureux,
- produire un effet relaxant sur l'animal,
- aider un animal à s'adapter à une situation nouvelle par exemple au moment de l'adoption, lors de l'arrivée d'un nouvel animal ou d'un enfant dans son environnement, lors d'un déménagement,
- réduire le marquage urinaire associé au stress chez un animal,
- réduire la malpropreté et les souillures d'un animal,
- améliorer les conditions de familiarisation d'un animal avec ses congénères,
- empêcher les griffades ou la destruction du territoire,
- diminuer les manifestations sonores telles que pleurs, aboiements,
- engendrer un comportement moins agressif, plus détendu et plus affectueux notamment avec leurs maitres chez un animal,
- faciliter le dressage et/ou l'éducation d'un animal,
la composition sémiochimique étant comprise dans un dispositif tel que défini précédemment comprenant :
• une matrice polymère,
• une composition sémiochimique,
• un agent de contrôle de la diffusion de la composition sémiochimique hors du dispositif,
• éventuellement un antioxydant,
• éventuellement un agent anti-UV,
• éventuellement un agent d'écoulement.

Préférentiellement, la composition sémiochimique apaisante est administrée à l'animal dans un dispositif comprenant :
- une matrice polymère,
- une composition sémiochimique, en particulier une composition sémiochimique apaisante, comprenant un mélange de dérivés volatils d'acide gras
- un agent de contrôle de la diffusion de la composition sémiochimique hors du dispositif.

De manière avantageuse, l'effet procuré par le dispositif est choisi parmi :
- assurer le bien-être d'un animal, dans son environnement,
- réduire le stress associé à la séparation chez un animal,
- réduire le stress associé à la solitude chez un animal,
- réduire le stress chez un animal avant et/ou pendant un transport,
- réduire le stress associé aux bruits tels que les orages ou les feux d'artifice,
- réduire le stress associé à une pathologie, un traitement médical, la visite chez un vétérinaire pouvant nécessiter une auscultation, voire une intervention chirurgicale,
- aider un animal à s'adapter à une situation nouvelle par exemple au moment de l'adoption, lors de l'arrivée d'un nouvel animal ou d'un enfant dans son environnement, lors d'un déménagement.

De préférence et selon tous les modes de réalisation qui précèdent, l'animal de compagnie est un chat ou un chien, préférentiellement, l'animal de compagnie est un chien.

La présente invention concerne dans un cinquième aspect l'utilisation d'un agent de contrôle de la diffusion d'une composition sémiochimique dans une matrice polymère pour permettre la libération d'une quantité relative plus élevée de composition sémiochimique et/ou pendant une durée plus longue et/ou la libération d'une quantité moyenne de composition sémiochimique équivalente sur successivement au moins deux intervalles de temps égaux, la libération de la quantité plus élevée et/ou pendant une durée plus longue et/ou la libération de la quantité moyenne de composition sémiochimique équivalente sur successivement au moins deux intervalles de temps égaux étant mesurée par rapport à la quantité mesurée lors de la diffusion de la même composition sémiochimique comprise dans une matrice polymère identique ne contenant pas l'agent de contrôle de la diffusion, où l'agent de contrôle de la diffusion est choisi parmi une argile, une diatomite et une zéolite ; ceci résulte en une diffusion d'une quantité relative, par rapport à la quantité totale dans le dispositif, plus élevée de la composition sémiochimique hors du dispositif par rapport aux dispositifs de l'art antérieur.

De préférence,
- l'agent de contrôle de la diffusion de la composition sémiochimique hors du dispositif est une argile, une diatomite ou une zéolite ;
- la composition sémiochimique est telle que décrite ci-avant, en particulier composée d'ester d'acides gras, de préférence d'esters méthyliques d'acides gras, telle que les compositions sémiochimiques **A** à **U** ;
- la matrice polymère est un copolymère thermoplastique d'acétate de vinyle et d'éthylène ; et
- l'utilisation de l'agent de contrôle de la diffusion de la composition sémiochimique hors du dispositif permet la libération d'une quantité relative plus élevée de composition sémiochimique et/ou pendant une durée plus longue et/ou la libération d'une quantité moyenne de composition sémiochimique équivalente sur successivement au moins deux intervalles de temps égaux par rapport aux dispositifs de l'art antérieur, en particulier, la libération d'une quantité plus élevée de composition sémiochimique et/ou pendant une durée plus longue et/ou la libération d'une quantité moyenne de composition sémiochimique équivalente sur successivement au moins deux intervalles de temps égaux dure pendant plus d'1 mois, de préférence pendant une durée d'environ 3 mois :
   - l'animal de compagnie est un chien.

De manière avantageuse, l'agent de contrôle de la diffusion de la composition sémiochimique hors du dispositif est choisi parmi les argiles possédant une structure en chaîne (chaînes de tétraèdres de silice semblables à la hornblende), en particulier les phyllosilicates tels que la wollastonite (par exemple vendu sous la dénomination casiflux F75), la bentonite, la kaolinite, la montmorillonite l'attapulgite, la sépiolite et la paligorskyte. De préférence, l'attapulgite, la sépiolite et la paligorskyte, encore préférentiellement, l'agent est la sépiolite.

Les dispositifs selon l'invention, quelle que soit leur forme, sont préparés par mélange des ingrédients le composant, ce mélange peut ensuite être mis en forme par différents moyens connu de l'homme du métier : en particulier, par extrusion, moulage ou encore impression 3D.

La fabrication par moulage, éventuellement par moulage par injection, nécessite de faire fondre le mélange d'ingrédients composant le dispositif, puis de l'introduire dans un moule ayant la forme du produit fini, ce moule peut être enduit d'un produit qui facilite le démoulage, le mélange fondu refroidit et se solidifie, le produit fini est ensuite démoulé. Le produit fini démoulé peut éventuellement être rectifié par coupage ou ponçage pour éliminer les imperfections.

La fabrication par impression 3D est de préférence mise en œuvre par « modelage par dépôt de filament en fusion » (FDM, Fused Deposition Modeling en anglais) ; cette technique consiste à déposer couche par couche un filament du mélange d'ingrédients fondu qui, en se superposant, donne forme à l'objet. La tête d'impression se déplace selon les coordonnées X, Y et Z (longueur, largeur et hauteur) transmises par un fichier 3D correspondant au modèle 3D de l'objet à imprimer.

Le principe de fonctionnement du procédé de fabrication par extrusion consiste à pousser le mélange d'ingrédients à travers une filière. La forme de la filière correspond à la section de la pièce finie.

La mise en œuvre de ce procédé d'extrusion comprend ainsi :
- le mélange d'ingrédients du dispositif ;
- l'extrusion de ce mélange dans une extrudeuse conduisant à un produit extrudé ;
- optionnellement, le calandrage du produit extrudé ;
- le découpage permettant d'obtenir le produit fini.

Selon un mode de réalisation illustratif, l'étape de mélange des ingrédients consiste à introduire le polymère (préférablement de l'EVA 9%) dans un mélangeur à socs par exemple, puis, d'ajouter la composition sémiochimique sous agitation. L'ajout peut par exemple être réalisé par pulvérisation, avec un jet de la composition sémiochimique. L'agent de contrôle de la diffusion de la composition sémiochimique hors du dispositif est ensuite ajouté (préférentiellement de la sépiolite). Le tout est mélangé. Ensuite, s'il est présent, l'agent d'écoulement est ajouté au mélange (préférentiellement 1% de silice colloïdale) et le tout est agité, puis tamisé. Par la suite, une autre dose de l'agent d'écoulement est ajoutée dans le tamis pour être tamisé (préférentiellement 0,4% de silice colloïdale). Enfin, la seconde moitié du polymère (préférablement de l'EVA 9%) est ajoutée dans le mélangeur à retournement et le tout est encore mélangé.

Le mélange d'ingrédients est ensuite introduit dans une extrudeuse qui peut être mono- ou bi-vis. Si nécessaire, la nappe de matière obtenue en sortie d'extrusion est calandrée afin d'obtenir les dimensions exactes requises ; le calandrage permet également un refroidissement de la nappe sortie de l'extrusion afin d'obtenir un produit solide.

Le produit extrudé se présente sous la forme d'un ruban qui est alors découpé pour obtenir le produit fini.

### FIGURES :

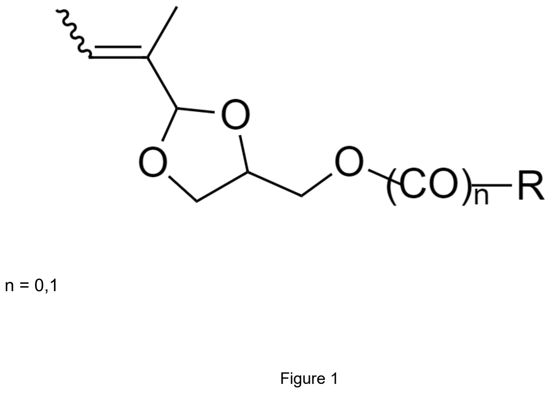

[Fig 1] : ester dérivé volatil d'acide gras
[Fig 2] : relargage des esters méthyliques d'acides gras au cours du temps (exemple 2A).

### EXEMPLES

### Exemple 1 : Préparation de colliers apaisant pour chien selon l'invention

### Exemple 1A

Le dispositif sous forme de collier de la présente invention est constitué d'un mélange comportant des actifs sémiochimiques (mélange **R** d'esters méthyliques d'acides gras comprenant du laurate de méthyle, du myristate de méthyle, du palmitate de méthyle, du stéarate de méthyle, de l'oléate de méthyle et du linoléate de méthyle), une matrice polymère de type éthylène-acetate de vinyle (EVA à 9%), un agent de contrôle de la diffusion de la composition sémiochimique hors du dispositif de type argile (montmorillonite 18,5%) et un agent d'écoulement (silice colloïdale à 1% puis à 1% séquentiellement).

### Préparation :

Une première étape consiste à introduire la moitié du polymère (EVA 9%) dans un mélangeur à socs, puis, d'ajouter le mélange d'esters méthyliques d'acides gras par pulvérisation sous agitation. La montmorillonite est ajoutée ensuite. Le tout est mélangé durant 30 secondes.

Une deuxième étape consiste à ajouter l'agent d'écoulement au mélange (1% de silice colloïdale), à mélanger durant 30 secondes, puis à tamiser le mélange. Par la suite, une autre dose de l'agent d'écoulement est tamisée dans le mélange (1% de silice colloïdale).

Enfin, la seconde moitié du polymère (EVA 9%) est ajoutée dans le mélangeur à retournement et le tout est encore mélangé pendant 3 minutes.

Ce mélange est ensuite mis en forme par extrusion.

### Exemple 1B

Le dispositif sous forme de collier de la présente invention est constitué d'un mélange comportant des actifs sémiochimiques (mélange **R** d'esters méthyliques d'acides gras comprenant du laurate de méthyle, du myristate de méthyle, du palmitate de méthyle, du stéarate de méthyle, de l'oléate de méthyle et du linoléate de méthyle), une matrice polymère de type éthylène-acetate de vinyle (EVA à 9%), un agent de contrôle de la diffusion de la composition sémiochimique hors du dispositif de type argile (sépiolite 15%) et un agent d'écoulement (silice colloïdale à 01,9 %).

### Préparation :

Dans une première étape, la sépiolite et la silice colloïdale sont mélangés dans un mélangeur à socs. Puis, le mélange d'esters méthyliques d'acides gras est ajouté sous agitation. Le tout est mélangé durant 20 secondes et tamisé. Le mélange est transféré dans un mélangeur à retournement contenant de l'EVA 9% et le tout est mélangé pendant 3 minutes.

Ce mélange est ensuite mis en forme par extrusion.

### Exemples 1C (lot D17190), 1D (lot D17191), 1E (lot D18001) et 1F (lot D17207) :

### Colliers 1C, 1D, 1E :

Le dispositif sous forme de collier de la présente invention est constitué d'un mélange comportant des actifs sémiochimiques (du laurate de méthyle, du myristate de méthyle, du palmitate de méthyle, du stéarate de méthyle, de l'oléate de méthyle et du linoléate de méthyle), une matrice polymère de type éthylène-acetate de vinyle (EVA à 9% pour le exemples 1C et 1E, EVA à 16% pour l'exemple 1D), un agent de contrôle de la diffusion de la composition sémiochimique hors du dispositif de type argile (sépiolite 15%), un agent d'écoulement (silice colloïdale à 1%, puis à 0,4% séquentiellement), un antioxydant (TBHQ 0,005%), et un agent anti-UV pour l'exemple 1E uniquement (Hostavin N30 P 1%).

### Collier 1F:

Le dispositif sous forme de collier de la présente invention est constitué d'un mélange comportant des actifs sémiochimiques (mélange **R** d'esters méthyliques d'acides gras comprenant du laurate de méthyle, du myristate de méthyle, du palmitate de méthyle, du stéarate de méthyle, de l'oléate de méthyle et du linoléate de méthyle), une matrice polymère de type éthylène-acetate de vinyle (EVA à 9%), un agent de contrôle de la diffusion de la composition sémiochimique hors du dispositif de type argile (sépiolite 15%), un agent d'écoulement (silice colloïdale à 1,4%), et un antioxydant (TBHQ 0,005%).

### Préparation :

Les exemples 1C, 1D, 1E et 1F ont été préparés selon le même mode de préparation.

La moitié de l'EVA a été introduit dans le mélangeur à socs. Par ailleurs, l'antioxydant TBHQ a été dissout dans le mélange d'esters méthyliques d'acides gras, et ce mélange a été pulvérisé sous agitation sur l'EVA selon un jet perpendiculaire à l'axe. La sépiolite comme agent de contrôle de la diffusion de la composition sémiochimique hors du dispositif a ensuite été ajoutée et le tout a été mélangé durant 30 secondes. Puis, 1% de silice colloïdale (+ agent anti-UV (Hostavin N30 P) pour l'exemple E) a été introduite en mélangeant encore durant 30 secondes. Le mélange a par la suite été tamisé dans un tamis de 1mm de maillage.

Une autre dose de silice colloïdale (0,4%) a ensuite été tamisée dans le mélange. Celui-ci a été transféré dans la seconde moitié de l'EVA, dans un mélangeur à retournement. Le tout a enfin été mélangé durant 3 minutes.

Ce mélange est ensuite mis en forme par extrusion.

### Récapitulatif des exemples 1A à 1F :

**[Table 1]**

| **Exempl e** | **Lot** | **Composition sémiochimique *** | **Matrice polymère** | **agent de contrôle de la diffusion de la composition sémiochimique hors du dispositif** | **Agent d'écoule ment** | **Agent anti-UV** | **Antioxy dant** |
|---|---|---|---|---|---|---|---|
| 1A | D1706 1 | Mélange R (5,21%) | EVA (VA 9%) | montmorillonite (18,5%) | silice colloïdale (2%) | non | non |
| 1B | D1706 6 | Mélange R (4,82%) | EVA (VA 9%) | sépiolite (15%) | silice colloïdale (1,9%) | non | non |
| 1C | D1719 0 | Mélange R (4,87%) | EVA (VA 9%) | sépiolite (15%) | silice colloïdale (1,4%) | non | oui (TBHQ 0,005%) |
| 1D | D1719 1 | Mélange R (4,87%) | EVA (VA 16%) | sépiolite (15%) | silice colloïdale (1,4%) | non | oui (TBHQ 0,005%) |
| 1E | D1800 1 | Mélange R (4,87%) | EVA (VA 9%) | sépiolite (15%) | silice colloïdale (1,4%) | oui (Hosta vin N30 P 1%) | oui (TBHQ 0,005%) |
| 1F | D1720 7 | Mélange R (4,87%) | EVA (VA 9%) | sépiolite (15%) | silice colloïdale (1,4%) | non | oui (TBHQ 0,005%) |

| | | | | | | | |
|---|---|---|---|---|---|---|---|
| *laurate de méthyle, myristate de méthyle, palmitate de méthyle, stéarate de méthyle, oléate de méthyle, linoléate de méthyle | | | | | | | |

### Exemple 2: Caractérisation du profil de relargage de la composition sémiochimique (FAME)

### Exemple 2A - Etude du relargage in vivo pendant 1 mois

Une étude comparative de différents colliers contenant une composition sémiochimique a été menée chez le chien afin de mesurer la cinétique de relargage de la composition et vérifier la bonne tolérance locale des colliers chez l'animal.
Pour ce faire, trois colliers de compositions différentes ont été testés : collier commercial témoin ADAPTIL^{®} lot P098663/099295, le collier préparé selon l'exemple 1C (lot D17190) et un collier préparé selon l'exemple 1D (Iot D17191).

Ainsi trois groupes de huit chiens ont chacun porté un des trois colliers durant quatorze jours ou durant vingt-huit jours (4 chiens par échéance et par groupe).

A l'issue de chacune des échéances, les esters méthyliques d'acides gras encore présents dans les colliers ont été dosés comme décrit à l'exemple 2A.

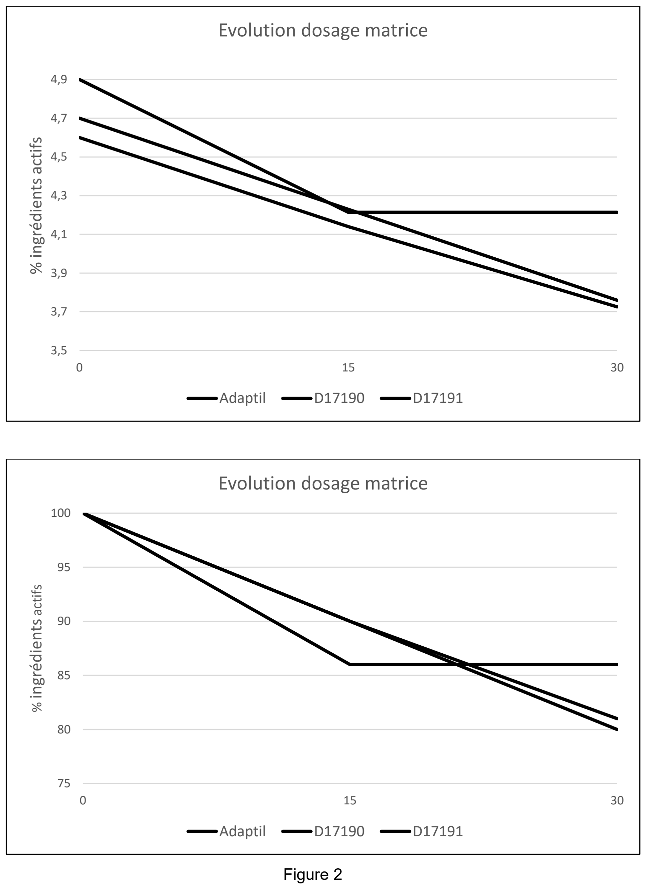

Les résultats du relargage des esters méthyliques d'acides gras au cours du temps sont présentés dans le tableau suivant ainsi que sur la figure 2:

**[Table 2]**

| | **T0** | **T14j** | **T28j** |
|---|---|---|---|
| Collier témoin | 100% | 86% | 86% |
| Collier 1C (EVA 9%) | 100% | 90% | 81% |
| Collier 1D (EVA 16%) | 100% | 90% | 80% |

Au bout de 14 jours, la quantité d'esters méthyliques d'acides gras relarguée n'est pas significativement différente entre le collier témoin et les deux colliers selon l'invention testés.

En revanche, au bout de 28 jours, les deux colliers selon l'invention testés contiennent moins d'esters méthyliques d'acides gras, indiquant que les colliers selon l'invention mettent à disposition plus d'esters méthyliques d'acides gras qu'à 14 jours, contrairement au collier témoin dont la quantité d'esters méthyliques d'acides gras relarguée ne varie pas significativement.
Entre T0 et T28j, la quantité d'esters méthyliques dans le collier selon l'invention décroît par ailleurs de manière régulière, de 10% par période de 14 jours, ce qui n'est pas le cas dans le collier témoin.

### Exemple 2B - Etude du relargage in vivo pendant 3 mois

Une étude comparative *in vivo* a été réalisée, sur un temps de trois mois avec pour objectif d'évaluer le temps pendant lequel un dispositif selon l'invention est capable de relarguer la composition sémiochimique apaisante.
Cette fois, le collier commercial témoin ADAPTIL^{®} lot P100736/101294 a été comparé au lot D18001 (sépiolite + EVA 9%) préparé selon l'exemple 1E.

Ainsi, seize chiens au total sont répartis en deux groupes et chacun a porté un des deux colliers durant 1 ou 3 mois (4 chiens par échéance et par groupe).

A l'issue de chaque échéance, les actifs ont été dosés en matrice et les résultats sont présentés dans le tableau suivant :

**[Table 3]**

| | **Collier 1E** | **Collier témoin** |
|---|---|---|
| T1M | 79% | 86% |
| T3M | 52% | 72% |

Les résultats de cette étude ont montré que :
- Le collier selon l'invention permet un relargage pendant au moins trois mois
- Le collier selon l'invention permet une mise à disposition plus importante de la composition semiochimique que le témoin pendant au moins trois mois.

### Exemple 3 : Etude de la stabilité de la composition pendant 3 mois

La stabilité d'une première série de formules a été testée sur trois mois pour comparer l'influence de l'agent de contrôle de la diffusion de la composition sémiochimique hors du dispositif (sépiolite ou montmorillonite) et du taux de vinyle acétate (VA) dans le copolymère EVA (9% ou 16%). Les formules ont été stockées sans emballage dans les enceintes climatiques.

**[Table 4]**

| Lot | Composition sémiochimique* | Matrice polymère | agent de contrôle de la diffusion de la composition sémiochimique hors du dispositif |
|---|---|---|---|
| D17126 | 5% mélange R | EVA (VA 9%) | montmorillonite (18,5%) |
| D17127 | 5% mélange R | EVA (VA 9%) | sépiolite (15%) |
| D17129 | 5% mélange R | EVA (VA 16%) | sépiolite (15%) |

**[Table 5]**

| | **25°C/60%RH** | | **40°C/75%RH** | |
|---|---|---|---|---|
| | **T1 mois** | **T3 mois** | **T15 jours** | **T1 mois** |
| D17126 (base montmorillonite / VA 9%) | 102.5% T0 | 94.9% T0 | 93.4% T0 | 92.6% T0 |
| D17127 (base sépiolite / VA 9%) | 94.6% T0 | 90.3% T0 | 93.8% T0 | 99.0% T0 |
| D17129 (base sépiolite / VA 16%) | 106.1% T0 | 100.3% T0 | 106.1% T0 | 108.2% T0 |

Les lots à base de sépiolite présentent des résultats homogènes tout au long de l'étude de stabilité qu'il s'agisse de formules avec EVA avec VA 9% ou 16%. Le lot à base de montmorillonite et d'EVA VA 9% présente une stabilité acceptable après 3 mois.
En ce qui concerne la première série, cette stabilité a montré que sur trois mois, les lots à base de sépiolite sont plus stables au niveau de la teneur en composition sémiochimique.

Puis, la stabilité d'une deuxième série de formules à base de sépiolite, EVA à 9% et plusieurs additifs (antioxydant, anti-UV, dispersant) a été testée. Les additifs ont notamment été introduits séparément afin d'étudier leur effet sur la stabilité. L'effet d'une concentration plus importante d'actif a également été étudié (5% ou 10%). Les formules ont été stockées sans emballage dans les enceintes climatiques. De plus, les formules conservées à 40°C/75%HR ont été exposées 8h par jour à la lumière UV pendant 1 mois de stockage.

**[Table 6]**

| **Lot** | **Compositi on sémiochim ique*** | **Matrice polymère** | **agent de contrôle de la diffusion de la compositi on sémiochim ique hors du dispositif** | **Agent d'écoulem ent** | **Agent anti-UV** | **Antioxyda nt** |
|---|---|---|---|---|---|---|
| D17161 | 10% mélange R | | Sepiolite (30%) | Silice colloidale (1,4%) | - | - |
| D17162 | 5% mélange R | EVA (VA 9%) | Sépiolite (15%) | | Hostavin ARO 8P (0.5%) | - |
| D17163 | 5% mélange R | | | | Hostavin N30P (0.5%) | - |
| D17164 | 5% mélange R | | | | - | TBHQ (0.005%) |

**[Table 7]**

| | **25°C/60%RH** | | **40°C/75%RH** | |
|---|---|---|---|---|
| | **T1 mois** | **T3 mois** | **T15 jours** | **T1 mois** |
| D17161 (FAME 10%) | - | 104.8% T0 | 100.5% T0 | 103.7% T0 |
| D17162 (0.5% Anti UV 1) | 91.5% T0 | 94.6% T0 | 93.0% T0 | 93.0% T0 |
| D17163 (0.5% Anti UV 2) | - | 101.8% T0 | 98.9% T0 | 102.4% T0 |
| D17164 (0.005% Anti-oxydant) | - | 98.9% T0 | 98.1% T0 | 92.6% T0 |

Pour la deuxième série, il a été mis en évidence que les colliers exposés pendant 8 heures par jour à la lumière UV sont stables. La présence d'un agent anti-UV ou d'un antioxydant est donc optionnelle.

### Exemple 4 : Etude in vivo : perception d'efficacité

Une étude de consommateurs a été réalisée au domicile de propriétaires de chiens, en France, notamment en évaluant l'efficacité d'un collier selon la présente invention en complément ou non à un collier antiparasitaire.

Des propriétaires de chiens ont alors été interrogés via des questionnaires sur leur perception de différents critères comportementaux de leur chien.

Ces chiens étaient au nombre de un dans leur foyer, sans cohabitation avec un chat, âgés de plus de trois mois, femelles et mâles confondus, la majorité d'entre eux n'étant pas castrés/stérilisés, aucun n'étant en gestation ou lactation, sans contrainte de sortie extérieure, ne présentant pas de problèmes de peau ou d'allergies de peau, n'étant pas traités pour troubles du comportement, mais présentant des troubles mineurs du comportement.

Le test à domicile des colliers a duré trois mois et les foyers ont été divisés en deux groupes de 75 chiens au total : un groupe de 45 chiens a reçu un collier lot D18101 (port du même collier sur trois mois), et le deuxième groupe de 30 chiens a reçu un collier lot D18101 et un collier antiparasitaire (port simultané des deux colliers sur trois mois). Dans chacun de ces groupes, 50% des chiens pesaient moins de 10kg et 50% des chiens pesaient plus de 10kg.

A l'issue des trois mois, on a constaté un impact positif des colliers sur les signes de stress ou d'anxiété pendant toute la période de port des colliers, alors qu'aucune différence significative de comportements n'a été mise en évidence entre les deux groupes de chiens. Il est aussi à noter que le port du collier du lot D18101 selon l'invention n'altère pas l'efficacité de l'action du collier antiparasitaire et inversement.

## Revendications

1. Dispositif pour relarguer de manière continue sur un animal ou dans l'environnement immédiat d'un animal une composition sémiochimique pour animaux de compagnie comprenant :
- une matrice polymère,
- une composition sémiochimique comprenant un mélange de dérivés volatils d'acide gras,
- un agent de contrôle de la diffusion de la composition sémiochimique hors du dispositif choisi parmi une argile, une diatomite et une zéolite.

2. Dispositif selon la revendication 1, le dispositif étant un collier.

3. Dispositif selon l'une des revendications précédentes, dans lequel l'agent de contrôle de la diffusion choisi parmi l'attapulgite, la sépiolite et la paligorskyte.

4. Dispositif selon l'une des revendications précédentes, dans lequel l'agent de contrôle de la diffusion de la composition sémiochimique hors du dispositif est présent dans le dispositif en proportions variant de 5 à 25%, avantageusement de 10 à 20%, de préférence environ 15% en masse par rapport à la masse totale du dispositif.

5. Dispositif selon la revendication précédente, dans lequel la matrice polymère est un copolymère thermoplastique d'acétate de vinyle et d'éthylène.

6. Dispositif selon l'une des revendications précédentes, dans laquelle la composition sémiochimique comprend un mélange de dérivés volatils d'acides gras choisis dans le groupe constitué de l'oléate de méthyle, du palmitate de méthyle, de l'azélate de méthyle, du pimélate de méthyle, du caprate de méthyle, du laurate de méthyle, du myristate de méthyle, du palmitoléate de méthyle, du linoléate de méthyle, du stéarate de méthyle, de l'arachidonate de méthyle, du n-butyrate de méthyle, de l'isobutyrate de méthyle, de l'α-méthylbutyrate de méthyle, du caproate de méthyle, du pivalate de méthyle, du γ-linoléate de méthyle, de l'eicosapentanoate de méthyle, du pentadécanoate de méthyle, du tridécanoate de méthyle, du docosahexanoate de méthyle, avantageusement choisi dans le groupe constitué de l'oléate de méthyle, du palmitate de méthyle, du laurate de méthyle, du myristate de méthyle, du linoléate de méthyle, du stéarate de méthyle et du pentadécanoate de méthyle.

7. Dispositif selon l'une des revendications précédentes, dans laquelle la composition sémiochimique est un mélange d'esters d'acides gras comprenant entre 32% et 38% d'oléate de méthyle, entre 1 et 3% de laurate de méthyle, entre 13 et 16% de stéarate de méthyle, entre 18 et 24% de linoléate de méthyle, entre 3 et 7% de myristate de méthyle et entre 18 et 24% de palmitate de méthyle, en poids par rapport au poids total de la composition sémiochimique.

8. Dispositif selon l'une des revendications précédentes, dans lequel la composition sémiochimique est comprise entre 0,1% et 25% en masse de la masse totale du dispositif, avantageusement de 1 à 10% en masse, plus avantageusement de 3 à 8% en masse, de préférence 5% en masse de la masse totale du dispositif.

9. Dispositif selon l'une des revendications précédentes, comprenant en outre un agent d'écoulement telle que la silice colloïdale et un ingrédient permettant d'améliorer la stabilité dans le temps de la composition sémiochimique tels qu'un antioxydant ou un agent anti-UV.

10. Composition sémiochimique présente dans un dispositif selon l'une quelconque des revendications 1 à 9, pour son utilisation pour prévenir les manifestations de stress et/ou réduire l'anxiété chez un animal de compagnie pendant une durée supérieure à 1 mois, plus avantageusement supérieure à 2 mois, de préférence pendant une durée d'environ 3 mois.

11. Composition sémiochimique présente dans un dispositif selon l'une quelconque des revendications 1 à 9 pour son utilisation selon la revendication 10, l'animal de compagnie étant un chien.

12. Utilisation d'un agent de contrôle de la diffusion d'une composition sémiochimique dans un dispositif selon l'une quelconque des revendication 1 à 2 et 4 à 9 pour permettre la diffusion régulière dans le temps et/ou la diffusion d'une quantité relative plus élevée de ladite composition sémiochimique comprenant un mélange de dérivés volatils d'acide gras hors de ladite matrice polymère, la diffusion régulière et/ou la diffusion d'une quantité relative plus élevée étant mesurée par rapport à la quantité mesurée lors de la diffusion de la même composition sémiochimique comprise dans une matrice polymère identique ne contenant pas l'agent de contrôle de la diffusion, **caractérisé en ce que** l'agent de contrôle de la diffusion est choisi parmi une argile, une diatomite et une zéolite.

13. Utilisation selon la revendication 12, dans laquelle l'agent de contrôle de la diffusion est un phyllosilicate choisi parmi l'attapulgite, la sépiolite et la paligorskyte.

14. Utilisation selon l'une des revendications 12 ou 13, dans laquelle la matrice polymère est un copolymère thermoplastique d'acétate de vinyle et d'éthylène.
